# EUROPEAN PATENT APPLICATION

(11) **EP 1 729 133 A1**
(43) Date of publication of application: **06.12.2006**
(21) Application number: 06114355.8
(22) Date of filing: 23.05.2006
(51) Int. Cl.: G01N 33/68

(54) **Transthyretin as marker for nephrotoxicity**

(30) Priority: 03.06.2005 EP 05104826; 21.03.2006 EP 06111456
(71) Applicant: F. HOFFMANN-LA ROCHE AG, 4070 Basel (CH)
(72) Inventor: Caprioli, Richard, Brentwood, TN 37027 (US); Ducret, Axel, 4052 Basel (CH); Meistermann, Helene, 68100 Mulhouse (FR); Ruepp, Stefan, Moorpark, CA 93021 (US)

(57) **Abstract**

Transthyretin was identified as a novel marker for assessing nephrotoxicity. Transthyretin accumulates in the kidney cortex in nephrotoxicity, where toxic effects characterized by marked degeneration/regeneration of proximal tubes are observed in an organism exposed to a toxic compound.

## Description

The present application relates to transthyretin accumulation in the kidney cortex as a biomarker for nephrotoxicity.

Transthyretin is a plasma protein that is not expressed in the kidney, and its detection in plasma has been associated with the presence of e.g. certain tumors, malnutrition or pancreatitis.

The present invention relates to in situ detection of transthyretin accumulation that was identified in regions of the kidney cortex where degenerative changes occur. Therefore, according to the present invention, the detection of transthyretin accumulation in the kidney cortex can be used to assess nephrotoxicity.

We establish here that one of the biomarkers modulated upon gentamicin administration was identified as transthyretin (prealbumin), a 13 kDa blood transporter protein. This finding was then confirmed by Western blot showing that this protein is significantly more abundant in kidneys from gentamicin-treated rats than in control rats.

Among the adverse effects for which drug candidates are being tested during their development is nephrotoxicity.

Nephrotoxicity is characterized by a marked degeneration/regeneration of proximal tubules (grade 4), which was clearly observed in rats treated with 100 mg/kg/day gentamicin for 7 days, thus confirming nephrotoxicity induced by the gentamicin treatment. Therefore, gentamicin treatment was used to identify novel markers for nephrotoxicity. Rat kidney thin sections were analyzed by MALDI IMS. The obtained spectra were analyzed and peaks ranked. A peak at mass m/z 12 959 was ranked in 1^{st} position in the cortex with an average signal-over-noise of 5 in gentamicin-treated rat kidney and completely absent in the controls.

Cortex proteins were micro-extracted from the cortex area and were fractionated by reverse phase chromatography. The fraction containing the peak at mass m/z 12 959 was identified by tandem MS as transthyretin.

The presence of transthyretin was shown *in situ* and in the HPLC fraction. Similarly, it was demonstrated that the m/z species 12959, which was identified by tandem mass spectrometry as transthyretin S₂₈-Q₁₄₆, a processed form of transthyretin, was only present in the mass spectrum of the gentamicin-treated sample *in situ* and in the HPLC fraction D10.

Thus, the present invention provides a method of assessing nephrotoxicity, comprising the steps of: a) providing a kidney sample from a subject suspected of suffering from nephrotoxicity;
b) determining the accumulation of transthyretin in the cortex of the kidney of the subject suspected of suffering from nephrotoxicity, as compared to a healthy subject;
wherein an increased accumulation of transthyretin in the kidney cortex of a subject suspected of suffering from nephrotoxicity, compared to a healthy subject, is an indication for the presence of nephrotoxicity.

Said subject can be a mammal. Said mammal can be a non-human mammal. Said non-human mammal can be a rat.

The accumulation of transthyretin in the kidney cortex is determined by any suitable method. One such method is immunohistochemistry using an antibody specific for transthyretin and suitable for immunohistochemistry. Methods of performing immunohistochemistry are well known to the skilled person. Another method is electrophoresis of a kidney cortex extract followed by detection of transthyretin, e.g. by Western Blotting using an antibody specific for the transthyretin protein or by mass spectrometry. A preferred method is the detection of transthyretin accumulation in the kidney cortex by MALDI imaging mass spectrometry (IMS) of a kidney section. This makes it possible to detect and localize the processed form(s) of the marker in the damaged kidney cortex.
Figure 1 Matrix spotting strategy on kidney thin section
Figure 2 Cluster analysis of the 7 most discriminating features in the cortex of control versus gentamicin-treated rat kidney
Figure 3 Average signal of m/z 12959 in the cortex of control and gentamicin-treated rat kidney
Fig 4 Identification strategy
Fig 5 Tandem mass spectrometric analysis of m/z 12959 A) Full mass spectrum. B) Deconvulated mass spectrum. C) Annotated tandem mass spectrum
Fig 6 Database search result
Fig 7 A) Western Blot analysis of the cytosolic fractions from kidney cortex and from HPLC fraction D10. B) MALDI MS analysis of rat kidney cortex and of HPLC fraction D10G/C: HPLC fraction D10 gentamicin/control; G/C: kidney cytosolic fraction gentamicin/control

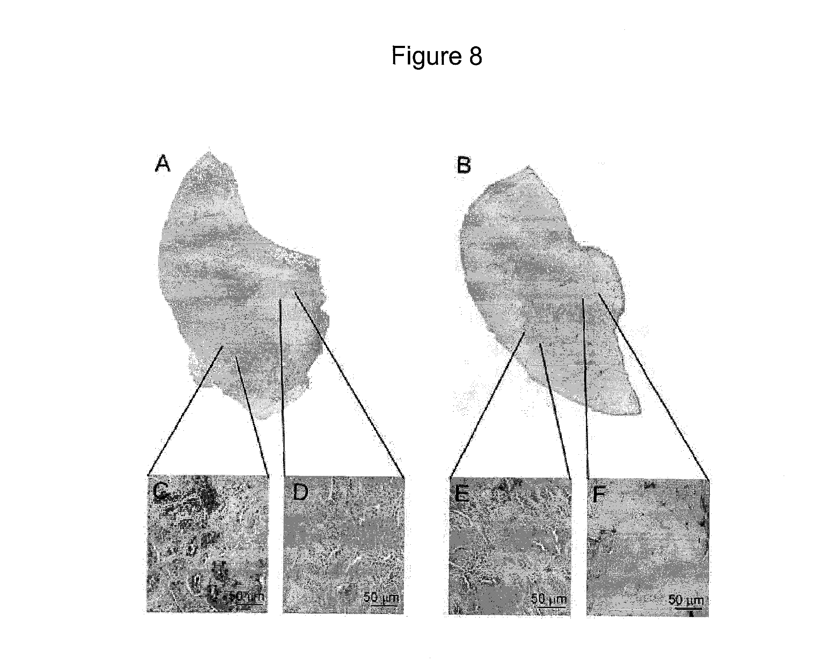

Fig 8: Immunohistochemical examination of kidney from control and gentamicin-treated rat. Scanned digitized images of whole kidney sections from a treated (A) and a control (B) rat show high levels of transthyretin immunoreactivity in the treated kidney cortex. High magnification photomicrographs from the cortex (C, E) vs. the medulla (D, F) reveal specific localization of TTR immunoreactivity in the cortex tubules of the treated kidney. Transthyretin immunoreactivity is visualized by the grey precipitate of diaminobenzidine-Ni counterstained with cresyl violet.

### Examples

### Example 1: Model system for nephrotoxicity

### Example 1a): Animal Treatment.

Permission for animal studies was obtained from the local regulatory agencies, and all study protocols were in compliance with animal welfare guidelines. Male HanBrl:
Wistar rats of approximately 12 weeks of age (300g ± 20%) were obtained from BRL, Füllinsdorf, Switzerland. The animals were housed individually in Macrolone cages with wood shavings as bedding at 20°C and 50% relative humidity in a 12-hr light/dark rhythm with free access to water and Kliba 3433 rodent pellets (Provimi Kliba AG, Kaiseraugst, Switzerland).

Animals were treated for 7 consecutive days with 100 mg/kg/day Gentamicin (dissolved in saline) or vehicle by subcutaneous (sc) injections and sacrificed 24 hours after the last application by CO₂ inhalation. Immediately preceding sacrifice, terminal blood samples for clinical chemistry investigations were collected from the retroorbital sinus under isofluoran anesthesia.

### Example 1 b): Histology.

Representative kidney samples were fixed in 10% neutral buffered formalin. All samples were processed using routine procedures and embedded in Paraplast. Tissue sections approximately 2-3 microns were cut and stained with hematoxylin-eosin (HE) or periodic acid-Schiff (PAS).

### Example 2: MALDI-IMS of rat kidney cortex treated with gentamicin

The differential protein expression was studied within the areas of the kidney (Cortex, Medulla, Papilla) to investigate whether the histopathologically-observed toxicity of gentamicin could be correlated by IMS. Several protein peaks in the cortex area were significantly modulated upon gentamicin administration.

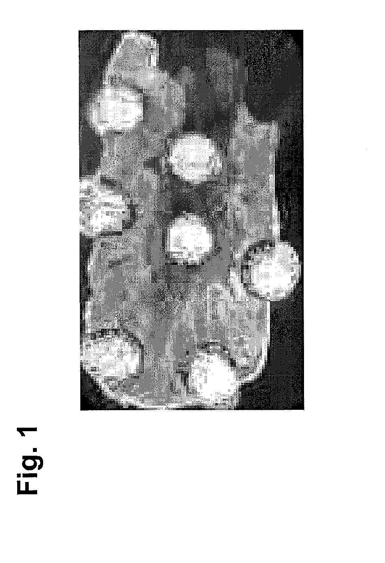

Marked degeneration/regeneration of proximal tubules (grade 4) was clearly observed in rats treated with 100 mg/kg/day gentamicin for 7 days. This finding was correlated to the analysis of rat kidney thin sections by MALDI IMS. 9 control and 9 gentamicin-treated rat kidneys thin sections (3 animals, 3 sections from each animal; the thin section was spotted with matrix as shown in Fig. 1) were subjected to mass spectrometric analysis as described below.

### Example 2 a): Sample Preparation for MALDI IMS.

Kidneys were dissected from rats, snap frozen on dry ice and stored at -80°C prior to further processing. 12 µm sections were obtained on a cryostat (LEICACM 3000, Leica Microsystems) at -18°C and deposited on ITO-coated conductive glass slides (Indium Tin Oxide 50 x 75 x 0.9 mm, Delta Technologies). Sample preparation were performed according to published procedure (Schwartz, S. A., Reyzer, M. L., and Caprioli, R. M. (2003) Journal of Mass Spectrometry 38, 699-708). Tissue sections were then fixed by immersion in ethanol bathes and let to dry 30 minutes under vacuum. During all the process the sections were stored as much as possible under vacuum. Deposition of MALDI matrix was done manually with a pipette. 200 nL of freshly prepared sinapinic acid solution (3,5-dimethoxy-4-hydroxycinnaminic acid, Fluka, 20 mg/ml in water/acetonitrile 1:1, 0.1% trifluoroacetic acid) was deposited onto the tissue. After crystallization, an additional 200 nL was layered onto the spots and the matrix was let to dry at room temperature. The sample was then kept under vacuum if not analyzed immediately by mass spectrometry.

### Example 2 b): Mass Spectrometry.

MALDI MS spectra were acquired using an UltraFlex MALDI ToF/ToF instrument (Bruker Daltonics) with a standard 337-nm N₂ laser operating at 50Hz and a laser spot size of 50 µm. The instrument was operated in positive linear mode (2kDa-30kDa) at constant laser power. A total of 8 times 100 laser shots were averaged for each manually deposited matrix droplets. Spectra were externally calibrated using a protein calibration standard solution (Insulin, Ubiquitin I, Cytochrom C, Myoglobin; Bruker Daltonics). Internal calibration was performed post-acquisition using redundant known peaks from the spectra (Hemoglobin α-chain, Thymosin β-4, Cytochrome c oxidase polypeptide VIIa L, Ubiquitin, Cytochrome c oxidase polypeptide VIb).

### Example 2 c): Data Analysis.

The spectra were processed using a combination of tools available commercially and developed in-house at Vanderbilt University. Baseline subtraction, normalization, peak detection, and spectral alignment were performed using the ProTS-Data software (Efeckta Technologies, Inc.). All spectra were baseline corrected to remove contributions of chemical noise to the signal. This step was performed by locally estimating the baseline and subtracting the spectrum from the raw data. For the present data set the software was set to consider a window width 10 times that of the peak width to account for the differences in resolution for different m/z regions. The baseline corrected spectra were normalized in intensity by scaling the spectra to a common total ion current. Peak detection was performed using ProTS-Data and a list of 13 common peaks was selected to be internal alignment points using a quadratic calibration function. The criterion used to select common peaks were that the peak must occur in greater than 80 % of the spectra to be aligned, must not have interfering or overlapping peaks, and the peaks must have a standard deviation of the observed centroid value less that 7 mass units. To avoid error caused by extrapolation during the alignment step, alignment peaks were chosen across the entire mass range of interest ranging from 2500 to 15000 mass units. The peak lists containing the centroid value and normalized intensity are exported to individual files.

The peak lists were binned according to their m/z value using an in-house program developed at Vanderbilt University. The exported MALDI-TOF MS peaks were aligned across samples by use of a genetic algorithm parallel search strategy (Yanagisawa, K., Shyr, Y., Xu, B. J., Massion, P. P., Larsen, P. H., White, B. C., Roberts, J. R., Edgerton, M., Gonzales, A., Nadaf, S., Moore, J. H., Caprioli, R. M., and Carbone, D. P. (2003) The Lancet 362, 433-439). Briefly, peaks were binned together such that the number of peaks in a bin from different samples is maximized while the number of peaks in a bin from the same sample is minimized. A series of mass windows or peak bins were generated that separated similar peaks across multiple spectra. The spectral features were ranked according to the extent of the observed difference in order to determine relevant biomarkers for gentamicin induced kidney toxicity using the Weighted Flexible Compound Covariate Method (WFCCM). This strategy, described by Shyr *et al.*(Shyr, Y., and KyungMann, K (2003) A Practical Approach to Microarray Data Analysis, ed D. Berrar*),* uses a combination of 6 different statistical tests to compute a ranking based on the difference observed between treated and control.

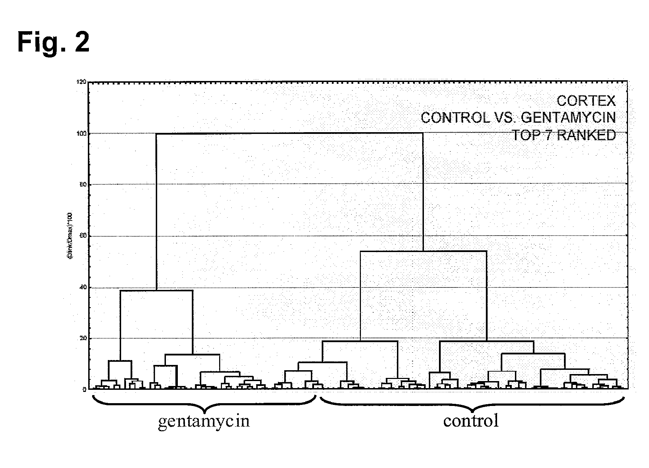

A simple data cluster analysis was successful in differentiating the control from the treated conditions. As expected, most of the peaks discriminating the control from the treated were found in the cortex. Table 1 shows the proteins that were statistically identified as being up or down regulated in the control versus the treated samples in the cortex. Fig 2 shows the cluster that can be obtained using the 7 top ranked peaks based on the WMA, the maximum average S/N and the p-value.

**Table 1: Cortex profiling: Biomarkers ranking for gentamicin-induced kidney toxicity**

| | Weighted Means Average | p-value | Average S/N (gent) | Average S/N (control) | Maximum Average S/N |
|---|---|---|---|---|---|
| 12959.23 | 1.5 | 2.E-14 | 5 | 0 | 5 |
| 6498.996 | -1.1 | 2.E-11 | 0 | 2 | 2 |
| 4497,792 | -0.9 | 9.E-11 | 0 | 5 | 5 |
| 6478.957 | 0.9 | 5.E-07 | 2 | 0 | 2 |
| 7083.364 | 0.8 | 2. E- 10 | 6 | 1 | 6 |
| 4107.083 | 0.8 | 9.E-07 | 5 | 0 | 5 |
| 5222.249 | -0.8 | 1.E-07 | 0 | 2 | 2 |
| 5096.886 | -0.7 | 2.E-11 | 5 | 11 | 11 |
| 4010.498 | -0.7 | 8.E-11 | 1 | 3 | 3 |
| 4141.907 | -0.7 | 2.E-10 | 1 | 3 | 3 |
| 5355.689 | -0.7 | 2.E-10 | 1 | 3 | 3 |
| 13004.71 | -0.7 | 6.E-09 | 0 | 3 | 3 |
| 17176.83 | -0.7 | 7.E-09 | 0 | 2 | 2 |
| 17100.68 | -0.7 | 1.E-06 | 0 | 2 | 2 |
| 6938.997 | 0.7 | 4.E-05 | 1 | 0 | 1 |
| 6912.956 | -0.6 | 2.E-09 | 6 | 10 | 10 |
| 3343.714 | -0.6 | 2.E-07 | 2 | 5 | 5 |
| 5301.652 | -0.6 | 4.E-07 | 0 | 3 | 3 |
| 4181.504 | -0.6 | 9.E-08 | 0 | 2 | 2 |
| 6080.035 | 0.6 | 6.E-04 | 1 | 0 | 1 |

Ranking by:
1) WMA
2) Maximum average S/N
3) p-value

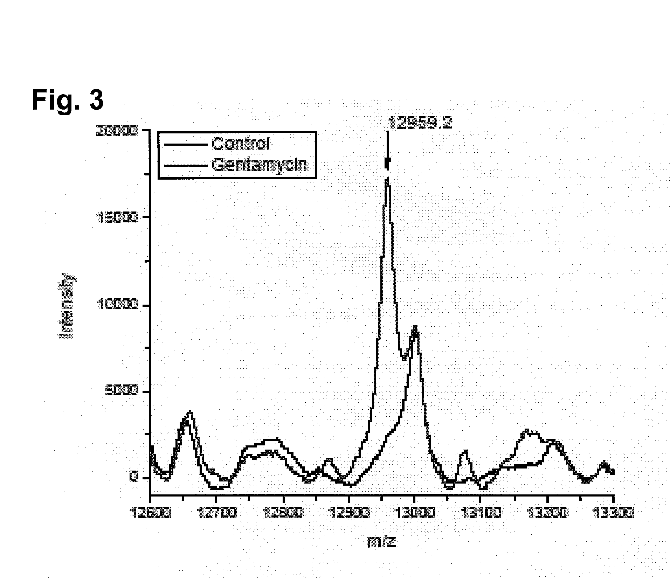

In this dataset, a peak at mass m/z 12 959 was ranked in 1^{st} position in the cortex with an average signal-over-noise of 5 in gentamicin-treated rat kidney and completely absent in the controls (Fig. 3). The doubly-charged species at m/z 6480 showed a similar behavior.

### Example 3: Microelution

A liquid micro-extraction was performed in the cortex area.

Protein micro-extraction was performed by directly pipetting up and down for a few seconds 1 µl of solvent (water/acetonitrile 1:1, 0.1% trifluoroacetic acid) on the cortex area of the kidney. This process was repeated several times on several samples to obtain a pooled sample volume of approximately 20 µl. The sample was then dried in a speed vac and resolubilized in water/acetonitrile 95:5, 0.1% trifluoroacetic acid.

The resulting protein mixture was fractionated by reverse-phase liquid chromatography.

Protein fractionation was performed using a standard Agilent 1100 series HPLC (Agilent Technologies) equipped with a 1 mm i.d. polymeric column (219TP5110, Vydac) operated at 50 µl/min. Solvent A was 0.1% trifluoroacetic acid in water and solvent B was 0.1% trifluoroacetic acid in acetonitrile. Proteins were eluted using the following program: the gradient was started after 5 minutes with 5%B up to 35% in 7 minutes, then to 60%B in 34 minutes, and then increased to 90%B in 0.5 minute and hold for 10 minutes. Fractions were collected every 30 sec by time immediately after the beginning of the run up to 48 minutes directly in a 96 well microtiterplate (Greiner bio-one). If necessary, a sample was fractionated over several HPLC runs and corresponding fractions were then combined for analysis.

The obtained LC fractions were surveyed by MALDI-TOF MS by spotting 1 µl of the fraction with 1 µl of 1 g/L sinapinic acid solution in water/acetonitrile 90:10, 0.1%TFA on a 384 MALDI Anchor target (Bruker Daltonics).

The presence of the peaks of interest was assessed by comparing the IMS-generated spectra with the LC-fractionated spectra.

### Example 3 a): Protein Identification.

The fraction containing the protein species of interest was then further analyzed by nano-ESI mass spectrometry and the proteins of interest were identified by tandem mass spectrometry.

For protein identification, the HPLC fraction containing the mass of interest was dried in a speed vac and resolubilized in water/acetonitrile 1:1 containing 1% formic acid for identification using an Applied Biosystems Q-Star Pulsar operated under standard operating conditions in nano-electrospray mode. One of the multiply charged species of the mass of interest was selected for tandem mass spectrometric analysis, its tandem mass spectrum was recorded and manually interpreted. Protein identification was performed with the Mascot MS/MS ion search program (Matrix Science, http://www.matrixscience.com) using the SwissProt database (release 46.6) and a tolerance of 1.0 Da for fragment and precursor masses.

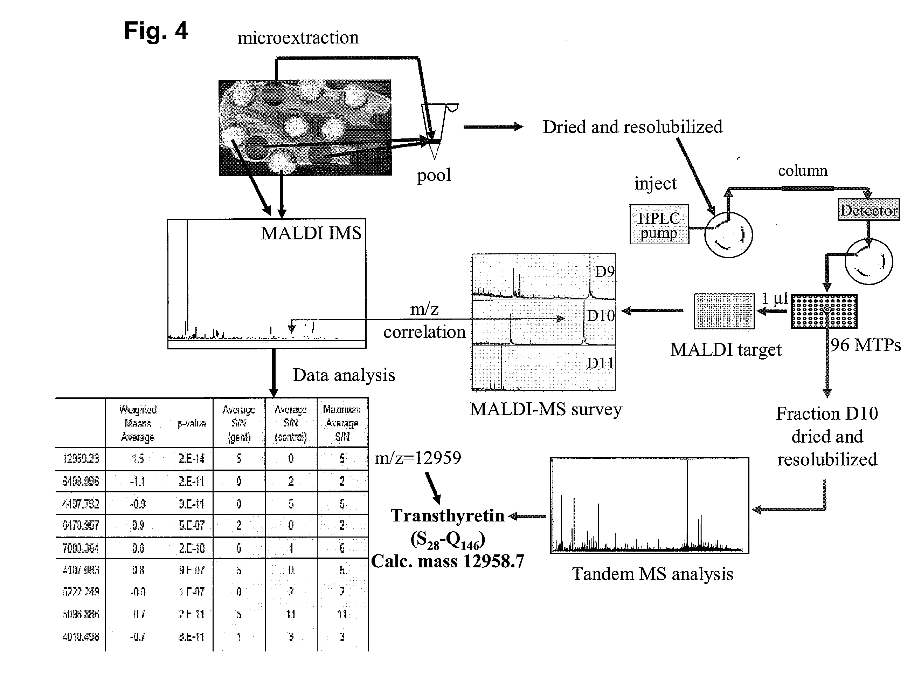

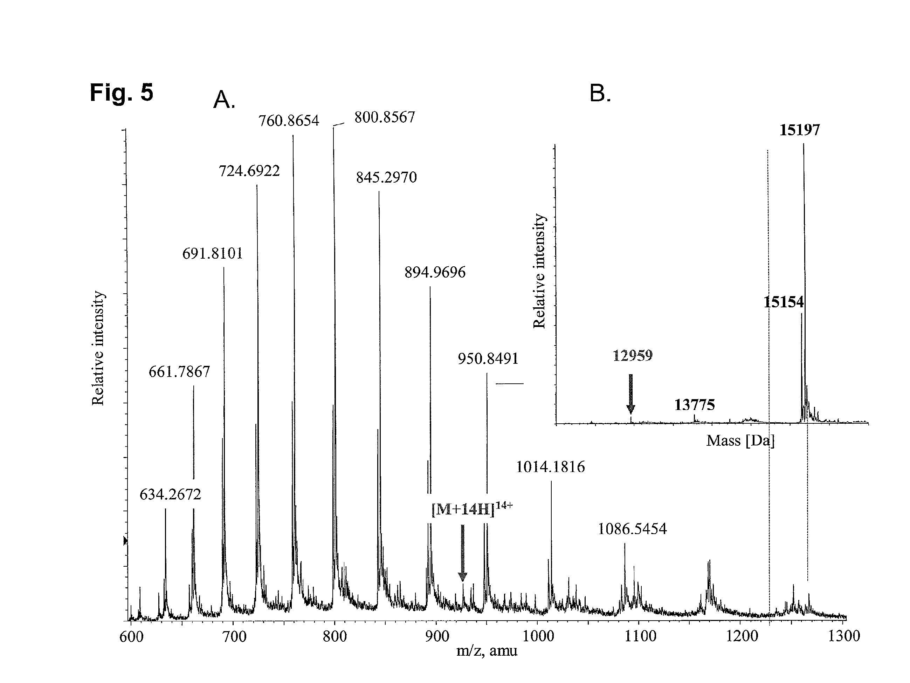

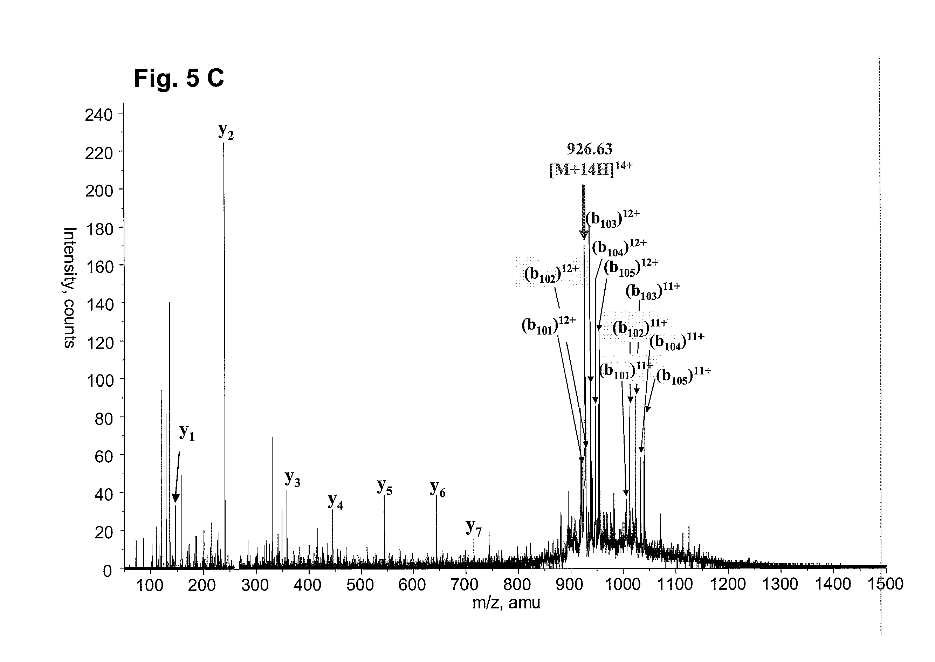

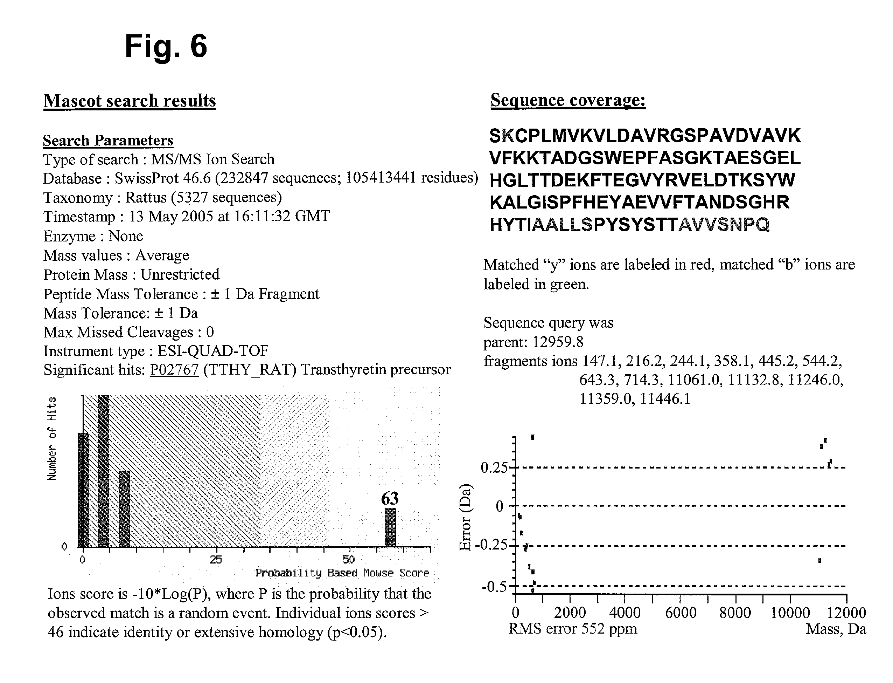

The strategy that was followed to identify this peak is illustrated in Fig. 4. Proteins were micro-extracted from the cortex area and were fractionated by reverse phase chromatography. A protein of m/z 12959 was found in the fraction D10 and could be successfully aligned with the species detected in the spectrum recorded directly from the tissue sample. The fraction was dried and reconstituted in 50% acetonitrile, 1% formic acid for tandem mass spectrometric analysis (Fig. 5). The generated mass spectrum could successfully be matched to transthyretin (sw:TTHY_RAT) from Ser28 to Gln146 (Fig. 6).

### Example 4: SDS-Polyacrylamide Gel Electrophoresis and Western Blot analysis of Tissue Extract and HPLC fraction.

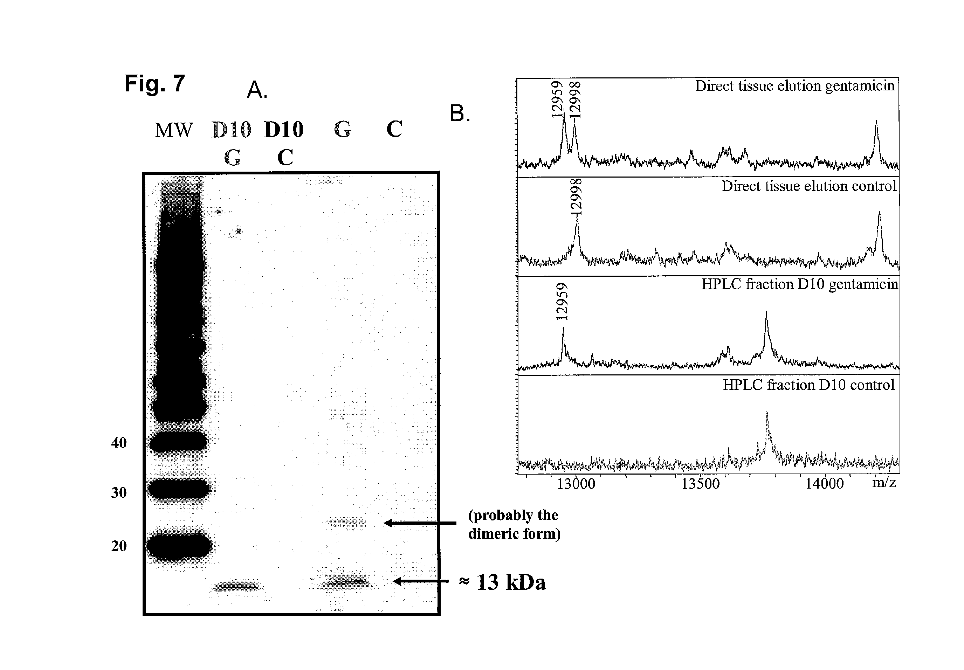

One of the biomarkers modulated upon gentamicin administration was identified as transthyretin (prealbumin), a 13 kDa blood transporter protein. This finding was confirmed by Western blot using an antibody highly specific for this protein showing that this protein was significantly more abundant in kidneys from gentamicin-treated rats than in control rats (Fig. 7).

Tissue extracts from control and treated rat kidneys were obtained by homogenization of a piece of cortex in a 10 mM Tris-HCI buffer pH 7.6 containing 250 mM sucrose and protease inhibitors (Roche Complete). The tissue extract was subjected to three successive centrifugation steps at 4 °C (10 min at 680g; 10 min at 10 000g; 1 h at 100 000g) whereas the pellets were discarded and the supernatant of the last centrifugation step was kept as the kidney cytosolic fraction.

1 µl of the kidney cytosolic fraction and of the relevant HPLC fraction were subjected to electrophoresis using a 4-20% Tris-Glycine SDS-PAGE gel (Invitrogen) and the proteins were subsequently transferred to a PVDF membrane. Blots were blocked in 5% milk in PBS containing 0.1% Tween 20 for 1 h and incubated overnight at room temperature with an anti-sheep prealbumin antibody (Abcam) in PBS containing 5% milk and 0.1% Tween 20. After washing with PBS containing 0.1% Tween 20, blots were incubated for 1 h with horseradish peroxidase conjugated with an anti-sheep IgG antibody (Silenus Laboratories). Antibody binding was detected using ECL western blotting reagents (Amersham).

Similarly, the m/z species 12959, which was identified by tandem mass spectrometry as transthyretin S₂₈-Q₁₄₆, was only present in the mass spectrum of the gentamicin-treated sample *in situ* and in the HPLC fraction D10.

## Claims

1. A method of assessing nephrotoxicity, comprising the steps of
a) Providing a kidney sample from a subject suspected of suffering from nephrotoxicity;
b) Determining the accumulation of transthyretin in the cortex of the kidney of the subject suspected of suffering from nephrotoxicity, as compared to a healthy subject,
wherein an increased accumulation of transthyretin in the kidney cortex of a subject suspected of suffering from nephrotoxicity, compared to a healthy subject, is an indication for the presence of nephrotoxicity.

2. The method of claim 1, wherein said subject is a non-human mammal.

3. The method of claim 2, wherein said non-human mammal is a rat.

4. The method of any one of claims 1 to 3, wherein said determining comprises measuring transthyretin accumulation in the kidney cortex by MALDI-IMS.


SEQUENCE LISTING

<110>  F. Hoffmann-La Roche AG

<120>  Transthyretin as a marker for nephrotoxicity

<130>  case 23145

<160>  2

<170>  PatentIn version 3.3

<210>  1

<211>  147

<212>  PRT

<213>  Rattus norvegicus

<220>

<221>  transthyretin

<222>  (1)..(147)

<223>  swissprot: locus TTHY_RAT, accession P02767

<400>  1

Met Ala Ser Leu Arg Leu Phe Leu Leu Cys Leu Ala Gly Leu Ile Phe
1               5                   10                  15

Ala Ser Glu Ala Gly Pro Gly Gly Ala Gly Glu Ser Lys Cys Pro Leu

            20                  25                  30

Met Val Lys Val Leu Asp Ala Val Arg Gly Ser Pro Ala Val Asp Val

        35                  40                  45

Ala Val Lys Val Phe Lys Lys Thr Ala Asp Gly Ser Trp Glu Pro Phe

    50                  55                  60

Ala Ser Gly Lys Thr Ala Glu Ser Gly Glu Leu His Gly Leu Thr Thr

65                  70                  75                  80

Asp Glu Lys Phe Thr Glu Gly Val Tyr Arg Val Glu Leu Asp Thr Lys

                85                  90                  95

Ser Tyr Trp Lys Ala Leu Gly Ile Ser Pro Phe His Glu Tyr Ala Glu

            100                 105                 110

Val Val Phe Thr Ala Asn Asp Ser Gly His Arg His Tyr Thr Ile Ala

        115                 120                 125

Ala Leu Leu Ser Pro Tyr Ser Tyr Ser Thr Thr Ala Val Val Ser Asn

    130                 135                 140

Pro Gln Asn

145

<210> 2

<211> 120

<212> PRT

<213> Rattus norvegicus

<220>

<221>  Ser 28 to Gln 146 processed form

<222>  (1)..(120)

<400>  2

Ser Lys Cys Pro Leu Met Val Lys Val Leu Asp Ala Val Arg Gly Ser
1               5                   10                  15

Pro Ala Val Asp Val Ala Val Lys Val Phe Lys Lys Thr Ala Asp Gly
            20                  25                  30

Ser Trp Glu Pro Phe Ala Ser Gly Lys Thr Ala Glu Ser Gly Glu Leu
        35                  40                  45

His Gly Leu Thr Thr Asp Glu Lys Phe Thr Glu Gly Val Tyr Arg Val
    50                  55                  60

Glu Leu Asp Thr Lys Ser Tyr Trp Lys Ala Leu Gly Ile Ser Pro Phe
65                  70                  75                  80

His Glu Tyr Ala Glu Val Val Phe Thr Ala Asn Asp Ser Gly His Arg
                85                  90                  95

His Tyr Thr Ile Ala Ala Leu Leu Ser Pro Tyr Ser Tyr Ser Thr Thr
            100                 105                 110

Ala Val Val Ser Asn Pro Gln Asn
        115                 120